# EUROPÄISCHE PATENTSCHRIFT

(11) **EP 3 128 965 B1**
(45) Veröffentlichungstag und Bekanntmachung des Hinweises auf die Patenterteilung: **02.05.2018**
(21) Anmeldenummer: 15721317.4
(22) Anmeldetag: 26.03.2015
(51) Int. Cl.: A61F 6/20, A61F 6/24

(54) **VORRICHTUNG ZUR KONTRAZEPTION ZUR ANWENDUNG BEIM MANN**
DEVICE FOR CONTRACEPTION FOR USE BY A MAN
DISPOSITIF DE CONTRACEPTION DESTINÉ À UNE APPLICATION CHEZ L'HOMME

(30) Priorität: 08.04.2014 CH 542142014
(43) Veröffentlichungstag der Anmeldung: 15.02.2017
(73) Patentinhaber: Pes Innovation AG, 9100 Herisau (CH)
(72) Erfinder: BIMEK, Clemens, CH-5503 Schafisheim (CH)
(74) Vertreter: Tompkin, Christine
(86) Internationale Anmeldenummer: PCT/IB2015/052224
(87) Internationale Veröffentlichungsnummer: WO 2015/155618

(56) Entgegenhaltungen:
- DE-C1- 19 909 427
- US-B1- 8 616 212

## Beschreibung

### Technisches Gebiet

Die Erfindung betrifft eine Vorrichtung zur Kontrazeption beim Mann, insbesondere eine Vorrichtung zum temporären Unterbruch des Spermaflusses innerhalb der Samenleiter (ductus deferents oder vas deferents) des Mannes.

### Stand der Technik

Für die Zeugungsregelung durch den Mann gibt es das bekannte, aber risikobehaftete Kondom und die nahezu endgültige, aber sichere, Vasektomie.
Die Umkehrung einer Vasektomie ist nur mit grossem Aufwand verbunden und nicht sicher reversibel. Deshalb wird sie meist auch erst nach erfüllter Kinderanzahl gewählt.
In letzter Zeit hat die Zahl der Männer zugenommen, die sich vor Jahren einer Vasektomie unterzogen haben und nun auf Grund geänderter Lebenssituationen diese wieder rückgängig machen wollen. Die Entwicklung von hormonell wirkenden Medikamenten, welche die Fertilität des Mannes beeinflussen sollen, ist nach Medienmitteilungen diverser Pharmakonzerne wegen Aussichtslosigkeit eingestellt worden.

Vorrichtungen zum temporären Unterbruch des Spermaflusses werden in die Samenleiter im Hodensack (Scrotum) des Mannes implantiert.

Das US-Patent 4,200,107 beschreibt einen zylinderförmigen Gefässverbinder, der um die Samenleiter angebracht wird. Das US-Patent 6,513,528 beschreibt ein in die Samenleiter einzubringenden Silicon-Zylinder. Um den Samenleiter-Verschluss wieder rückgängig zu machen, ist jeweils in beiden Fällen eine weitere Operation notwendig.
Patentanmeldung PCT WO 2010/047644 A1 beschreibt eine sehr aufwendige technische Lösung, bei welcher der Verschluss der samenführenden Kanäle mittels einer zu implantierenden, die Kanäle abdrückende Manschette erfolgt, welche durch eine ebenfalls zu implantierende Pumpeinrichtung bedient wird. Diese Pumpeinrichtung wird von aussen durch die Haut des Anwenders mit Energie versorgt und gesteuert. Dazu sind zusätzlich eine Fernbedienung und ein induktiver Energieüberträger notwendig. Die Implantation ist mit hohem Aufwand verbunden, indem unter anderem zur Schmerzausschaltung mindestens eine Spinalanästhesie notwendig ist. Dies führt zu hohen Operationskosten und einem erhöhten gesundheitlichen Risiko für den Patienten. Die dafür benötigte Vielzahl der verschiedenen mechanischen, hydraulischen und elektronischen Komponenten erhöht zudem das Risiko technischen Versagens, und es entstehen weitere Energiekosten für den Betrieb der Vorrichtung.

Das Patent DE 19909422 C1 stellt den nächstliegenden Stand der Technik dar und beschreibt ein Ventil, welches in die Samenleiter des Mannes implantiert wird und mittels eines Schaltgriffes durch den Hodensack vom Anwender ertastet und so geöffnet oder geschlossen werden kann. Sie sind derart konstruiert, dass es dem Anwender möglich ist, seine Zeugungsfähigkeit selbst von aussen und ohne weitere chirurgische Eingriffe selbst zu beeinflussen. US 8,616,212 offenbart ein Ventil, das in einem Samenleiter oder einem Eileiter einsetzbar ist und der Geburtenkontrolle dient. Das Ventil kann manuell mit einem Dreharm geöffnet und geschlossen oder durch Fernbedienung mittels einem Solenoid und Radioempfänger betätigt werden. Eine Variante weist am Dreharm Magnete auf, die mittels einem Magnetfeld bewegt werden können. Beim Schliessen des Ventils wird ein Durchgang für den Fluss von Spermien bzw. von einer Eizelle abgesperrt.

### Beschreibung der Erfindung

Der vorliegenden Erfindung liegt nun die Aufgabe zugrunde, eine Vorrichtung zur Absperrung des Samenleiters eines Mannes zwecks Kontrazeption zu schaffen, das im Vergleich zu solchen Vorrichtungen des Standes der Technik, insbesondere bezüglich der Ventilschaltung verbessert ist.

Diese Aufgabe wird durch eine Vorrichtung mit den Merkmalen des Patentanspruchs 1 gelöst.

Die erfindungsgemässe Vorrichtung wird nachfolgend als Samenleiterventil bezeichnet.
Es wird ein implantierbares Samenleiterventil offenbart zur Kontrazeption beim Mann oder männlichen Tier zur Regulierung des Spermienflusses im Samenleiter, lateinisch ductus deferents oder Vas deferents, innerhalb des Scrotums. Es umfasst ein Ventil und zwei Ventilanschlussstücke, die jeweils an das testikuläre und abdominale Ende eines vorgängig durchtrennten Samenleiters befestigbar sind, wobei das testikuläre Samenleiterende jenes Ende aus den Hoden, lateinisch Testis kommt und das abdominale Ende zum Unterleib, lateinisch Abdomen führt. Das Ventil weist einen manuellen Schalter auf, mit dem einerseits der Schaltzustand des Ventils von aussen durch Abtastung oder Palpation feststellbar ist und anderseits zwischen einem geöffnetem und geschlossenem Zustand veränderbar ist.
Erfindungsgemäss weist das Samenleiterventil einen Durchgangskanal und mindestens einen Abflusskanal auf, wobei der Durchgangskanal im geöffneten Zustand des Ventils vom Ventilanschlussstück am testikulären Samenleiter zum Ventilanschlussstück am abdominalen Ende des Samenleiters führt. Im geschlossenen Ventilzustand ist das Ende des Durchflusskanals verschlossen, das dem abdominalen Samenleiterende zugewandt ist. Der mindestens eine Abflusskanal führt im geschlossenen Zustand des Ventils von dem
In einer weiteren Ausführung der Erfindung weist das Samenleiterventil einen Entsicherungsbolzen auf, der zum Öffnen des Ventils zunächst betätigt werden muss.

Die Erfindung hat den Vorteil, dass im geschlossenen Zustand des Ventils nur das abdominale Samenleiterende verschlossen ist, während das testikuläre Samenleiterende offen ist und der Samentransport von diesem Ende ungehindert weiter gehen kann. Zudem ermöglicht der Abflusskanal oder die mehreren Abflusskanäle den Abfluss von aus dem testikulären Samenleiterende austretenden Spermien, die somit aus dem Ventil und dessen Gehäuse strömen und in den Körper des Mannes gelangen können. Es entsteht damit aufgrund des erfindungsgemässen Ventils kein Stau der austretenden Spermien im Bereich des Nebenhodens. Spermien gelangen stattdessen in das Gewebe im Hodensack und werden dort von körpereignen Mechanismen abgebaut. Nach heutiger Erkenntnis hat dies keine pathologischen Auswirkungen.

Aufgrund des weggefallenen Risikos eines Samenstaus kann das erfindungsgemässe Samenleiterventil an beliebiger Stelle im Bereich des Hodensacks im Samenleiter implantiert werden. Es ist nicht mehr unbedingt notwendig, das Implantat so nahe dem Epididymis zu platzieren. Dies hat den Vorteil, dass das Implantat vom Chirurgen an einer Stelle implantiert werden kann, die dem Patienten am besten entspricht.
Die Erfindung ist ein passives Implantat innerhalb des Samenleiters (Ductus deferens) im Hodensack (Scrotum) des Mannes.
Das Implantieren des Ventils bedarf nur einer einfachen, risikoarmen, kostengünstigen, ambulanten Operation mittels Lokalanästhesie ähnlich einer Vasektomie, welche von jedem geschulten Urologen vorgenommen werden kann. Wie bei der Vasektomie werden die Samenleiter durchtrennt und die so entstandenen beiden Enden des Samenleiters auf die dafür vorgesehenen Anschlussstücke des Ventils geschoben und fixiert. Das Samenleiterventil ist somit mit den an ihm befestigten Samenleiter und den Hoden im Hodensack frei beweglich. Das Ventil kommt immer paarweise zum Einsatz, da in der Regel auch zwei Hoden (Testis) vorhanden sind.

Das Ventil ist derart konstruiert, dass der Schaltzustand des Ventils, also geöffnet oder geschlossen, vom Anwender selbst und ohne weiteren chirurgischen Eingriff durch Ertasten (Palpation) von aussen, durch die weiche Haut des Hodensacks hindurch, festgestellt und bei Bedarf durch Betätigung der Schaltwippe geändert werden kann. Beim Betätigen rastet die Mechanik jeweils exakt, sicher und spürbar in eine jeweilige Endstellung ein: Geöffnet oder geschlossen. Dabei sind in den geöffneten bzw. geschlossenen Ventilstellungen beispielsweise die Flächen der Schaltwippe bündig mit der Fläche des Ventilkörpers.

Das Ventil ist richtungsgebunden. Das heisst, dass bei der Implantation darauf geachtet werden muss, dass das abdominale Ende des Samenleiterventils auch an das abdominale Ende des Samenleiters befestigt wird und im gleichen Sinn auch das testikuläre Ende des Samenleiterventils mit dem testikulären Ende des Samenleiters verbunden wird.
Dazu wird wie bei allen technischen Ventilen üblich, eine Kennzeichnung der Flussrichtung mittels Pfeil angebracht. Der angebrachte Pfeil zeigt also immer in die natürliche aufsteigende Flussrichtung der Spermien in Richtung Unterleib des Menschen, lateinisch abdominal.

Durch Ertasten ist feststellbar, ob das Ventil geöffnet oder geschlossen ist.
Das Ventil ist geöffnet, wenn die auf der abdominalen Seite des Ventils liegende Fläche der Schaltwippe bündig zum Ventilkörper liegt und die hodenseitige oder testikuläre Kante der Schaltwippe aus dem Ventilkörper herausragt.
Das Ventil ist geschlossen, wenn die hodenseitige oder testikuläre Fläche der Schaltwippe bündig mit dem Ventilkörper liegt und die abdominale Kante der Schaltwippe aus dem Ventilkörper herausragt.

Zum Schliessen des Ventils wird die auf der zum Hoden zeigenden Seite des Ventils hochstehende Schaltwippe mit einer abrollenden Bewegung Richtung Hoden geschoben. Dadurch richtet sich die zum Körper oder zum Abdomen zeigende Kante der Schaltwippe auf und die Ventilstellung rastet fix ein.

Zum Öffnen des Ventils wird der Entsicherungsbolzen an dem unteren zu den Hoden zeigenden Ecke des Ventils gedrückt und die Schaltwippe mit einer abrollenden Bewegung in Richtung des zum Körper führenden Samenleiters geschoben. Dadurch erhebt sich die Schaltwippe auf der zum Hoden führenden testikulären Seite des Ventils.

Selbst im geschlossenen Zustand hat man wie bei der Vasektomie eine nahezu vollständige Ejakulation, da nur der Anteil, etwa 3-5 Vol. %, aus den Hoden fehlt. Das Ertasten und Umschalten des Ventils geht am besten, wenn der Hodensack einen weichen ausgedehnten Zustand hat. Der Hodensack dient der Temperaturregelung für die Hoden. Diese benötigen für eine optimale Spermiogenese eine Temperatur von etwa 3°C unterhalb der Körpertemperatur des Menschen. Bei kalter Umgebungstemperatur zieht sich der Hodensack zusammen, um die Hoden zu wärmen. Bei warmen bis heissen Bedingungen wird er weich, dehnt sich aus, und vergrössert seine Fläche, um die Hoden zu kühlen. Deshalb sorge der Anwender vor einem gewünschten Umschalten des Samenleiterventils für ein Erwärmen der Hodenregion.
Die gewählte Schaltstellung sollte logischerweise bei beiden Ventilen gleich sein. Das Samenleiterventil ist im Hodensack von aussen nicht erkennbar, da die Samenleiter immer von der Rückseite des Hodens weg verlaufen.

Die Erfindung schafft ein einfaches und sicheres Mittel, mit dem ein Mann jederzeit selbst bestimmen kann, ob er in einer gegebenen Lebenssituation Vater werden möchte. In einer heterosexuellen Partnerschaft ist er allein in der Lage und kann dafür Sorge tragen, ob, wann und in welchem zeitlichen Abstand er ein Kind zeugen möchte. Dies ist ihm mit der Erfindung ermöglicht, indem er einfach den erfindungsgemässen Schalter umlegt. Er muss keine Medikamente oder Hormone nehmen und es entstehen ihm keine permanent anfallenden Kosten. Seine Partnerin braucht ihre Gesundheit durch Verwendung bestehender Kontrazeptiva nicht zu riskieren. Der Mann muss dafür nie auf Sex verzichten und oder irgendwelche Utensilien verwenden oder dafür bereithalten müssen. Bei der hiermit offenbarten Erfindung handelt es sich um ein neuartiges Medizinprodukt zur männlichen Kontrazeption und müsste oder kann deshalb korrekterweise als Kontragenerativum bezeichnet werden. Diesen Begriff gibt es noch nicht in der Nomenklatur. Schon dies alleine zeigt, dass in Fachkreisen davon ausgegangen wird, dass die Geburtenregelung Frauensache ist. Denn für die Frau gibt es unzählige Möglichkeiten der Kontrazeption. Die Anwendung dieser Mittel ist aber immer mit Risiken und Nebenwirkungen verbunden. Die hiermit beschriebene Erfindung stellt somit eine Alternative zu den bisherigen Geburtenregelungen dar. Sie ermöglicht es dem Mann die Geburtenregelung allein zu übernehmen. Aufgrund der Einfachheit, der hohen Sicherheit, den geringsten Nebenwirkungen und Risiken und geringen Kosten kann sie allgemein angewendet werden.

Weitere Ausführungen und Details der Erfindung sind Gegenstand weiterer abhängiger Patentansprüche. Die Ausführungsbeispiele sowie ihre Vorteile sind in der nachfolgenden Beschreibung anhand der Figuren näher erläutert.

### Kurze Beschreibung der Figuren

Fig. 1 zeigt einen schematischen Querschnitt durch die Genitalien des Mannes mit der Position des erfindungsgemässen Samenleiterventils im Samenleiter im Bereich des Hodensacks.
Figur 2 zeigt die Ansicht des erfindungsgemässen Samenleiterventils in geöffneter Stellung, der an den Enden eines durchtrennten Samenleiters angeschlossen ist. Die Flussrichtung der Spermien durch das Samenleiterventil vom testikulären Samenleiterende zum abdominalen Samenleiter ist mit Pfeilen und "Flow" gekennzeichnet.
Figur 3 zeigt das erfindungsgemässe Samenleiterventil an den Samenleiterenden angeschlossen wie in Figur 2, jedoch in geschlossener Stellung. Die Flussrichtung der Spermien durch den testikulären Samenleiter bis zum Samenleiterventil ist durch die Pfeile und "Flow" bezeichnet. Der Spermienfluss durch den abdominalen Samenleiter ist durch die Schaltwippe in geschlossener Stellung unterbrochen.
Figur 4 zeigt einen vertikalen Längsschnitt des Samenleiterventils im geöffneten Zustand.
Figur 5 zeigt einen vertikalen Längsschnitt des Samenleiterventils im geschlossenen Zustand.
Figur 6 zeigt ein Ventilanschlussstück des Samenleiterventils zum Einstecken in einen Samenleiter.
Figur 7 zeigt das in einen Samenleiter gesteckte Ventilanschlussstück.
Figur 8 zeigt den Samenleiter mit einem aufgeweiteten Ventilanschlussstück.
Figur 9 zeigt zwei Ventilkappenhälften (4) und deren Einbindung mit dem Samenleiter- Ventilanschlussstück- aus Figur 6-8.
Figur 10 zeigt den Zusammenbau der in Figur 9 entstandenen Kombination von Samenleiter- Ventilanschlussstück und Ventilkappenhälften mit dem Samenleiterventil aus Figur 2 und 3.

In den Figuren sind für dieselben Elemente jeweils dieselben Bezugszeichen verwendet worden und erstmalige Erklärungen betreffen alle Figuren, wenn nicht ausdrücklich anders erwähnt.

### Ausführungsbeispiele der Erfindung

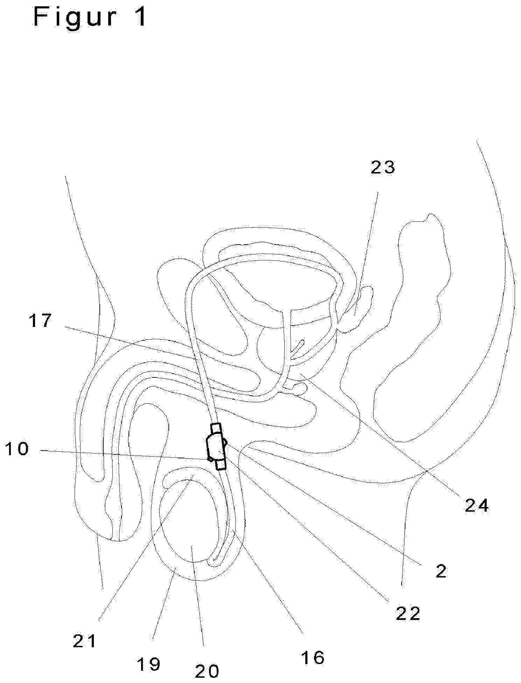

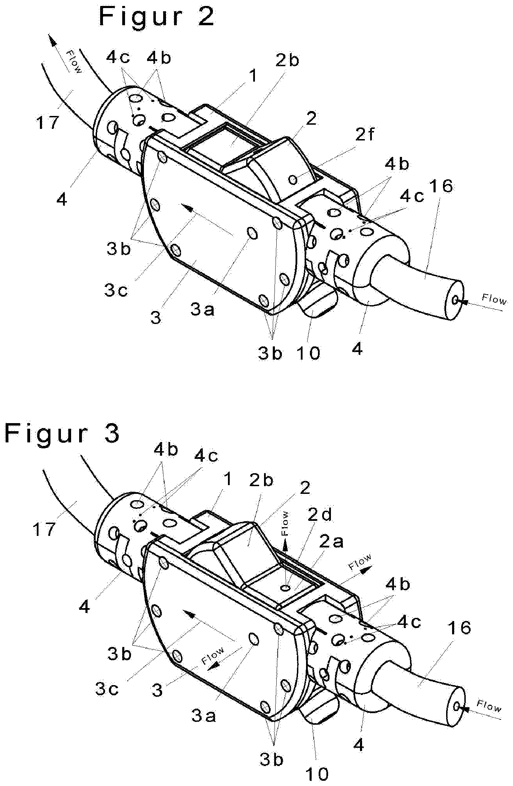

Das erfindungsgemässe Samenleiterventil 22 lässt sich beim Mann im Scrotum 19 im Samenleiter 16, 17 implantieren wie in der Figur 1 dargestellt. Der vom Hoden 20 verlaufende Samenleiter wird dazu zunächst durchtrennt, wobei das Ende am testikulären Teil des Samenleiters 16 sowie das Ende am abdominalen Teil des Samenleiters 17 nicht verschlossen werden, wie es etwa bei einer Vasektomie getan wird. Das Samenleiterventil 22 ist in der Darstellung zwischen dem Nebenhoden oder Epididymis 21 und der Bläschendrüse (Glandulae vesiculosa) 23 jedoch noch im Bereich des Hodensacks (Scrotums) 19 implantiert. Sie braucht nicht zwingend nahe dem der Epididymis 21 zu liegen. Das Samenleiterventil 22 besteht aus einem Ventilkörper 1 mit Seitenwänden 3, an den Enden der testikulären und abdominalen Samenleitern 16 und 17 angeschlossenen Ventilanschlusstücken, sowie einer Schaltwippe 2 und einem Entsicherungsbolzen 10. Das Samenleiterventil ist wie die Hoden und die Samenleiter im Hodensack im gewissen Mass frei beweglich, entsprechend der körperlichen Betätigung jeglicher Art des Mannes. Wie in den Figuren 2-5 dargestellt, besteht die Erfindung aus einem Ventilkörper 1, welcher der Aufnahme von seitlich und einander gegenüberliegend angebrachten, in den Figuren 2 bis 5 rechts und links dargestellten Ventilanschlussstücken 9 mit Ventilkappen 4, sowie der Schaltwippe 2 und den übrigen mechanischen Teilen dient. Der Ventilkörper 1 hat beispielsweise die Form eines Quaders mit allseitig, teilweise stark, abgerundeten Ecken und Kanten. Die Abrundungen vermeiden eine Traumatisierung des umschliessenden Gewebes. Der Ventilkörper 1 hat beispielsweise eine Länge von etwa 18 mm, eine Höhe von 10 mm und eine Tiefe von etwa 7 mm. Aber auch Abmessungen von bis zu 50 % grösser sind denkbar. Je kleiner das Ventil ist, desto grösser ist der Tragekomfort. Jedoch verringert sich dann die Bedienfreundlichkeit der Schaltwippe 2 und umgekehrt. Die angegeben Dimensionen sind im Selbstversuch erprobt und haben sich als guter Kompromiss bestätigt.
Die meisten Teile bestehen vorzugsweise aus Implantat-Kunststoff wie (z.B. PEEK) und werden in Mikrospritzgusstechnik und oder CNC-frästechnisch bearbeitet. Die Ventilanschlüsse 9 können aus Metall-Legierungen, wie zum Beispiel Titanlegierungen (z.B. Nitinol) oder einem geeigneten Implantat-Stahl (z.B. Werkstoff 1.4441/316 LVM), oder aus Kunststoff oder aus einer Kombination beider Werkstoffarten gefertigt sein. Es kann aber auch das gitterförmige Röhrchen 9c zum Beispiel aus Metall gefertigt sein und mittels Spritzguss in die zum Beispiel aus Kunststoff bestehenden Teile 9b und 9a eingebunden werden. Die benötigten Druckfedern können aus Implantat- Federstahl oder Platin-Legierungen gefertigt werden.

Der Ventilkörper 1 besitzt im oberen Drittel in Längsrichtung einen Durchgangskanal 1a von etwa 0,7 mm Durchmesser, der sich durch die Schaltwippe 2 sowie die Ventilanschlusstücke 9 erstreckt und durch den im geöffneten Zustand des Ventils der Spermienfluss stattfinden kann. An den Stirnseiten des quaderförmigen Ventilkörpers, axial zum Durchgangskanal 1a, befinden keilförmige Fräsungen 1d und kreisbahnförmige Fräsungen 1e für die Aufnahme der Ventilkappen 4 und Stufenbohrungen 1b zur Aufnahme der Ventilanschlussstücke 9 während der Implantation. Quer zum Durchgangskanal 1a und von der grossen Planfläche 3 des Ventilkörpers her befindet sich eine, zum Durchgangskanal 1a senkrecht stehende, grosse Stufenbohrung, beispielsweise von 12 mm Durchmesser und einer Tiefe von 5 mm. In der gleichen Achse und im Grund der grossen Stufenbohrung befindet sich eine kleinere Sacklochbohrung, beispielsweise von 2 mm Durchmesser und einer Tiefe von 0,7 mm. Diese kleine Sackbohrung dient der Aufnahme und der drehbaren Lagerung der Schaltwippe 2. Die grosse Stufenbohrung durchschneidet die obere Kante des Ventilkörpers, wodurch die montierte Schaltwippe 2 aus dem Ventilkörper herausragt. Im unteren Bereich der Stufenbohrung befinden sich in der Bohrungswand zwei gleichförmige Aussparungen 1c, die spiegelsymmetrisch zueinander stehen und jeweils mindestens eine Kreissegmentform aufweisen. Sie dienen als Führung und Anschlag einer gefederten Radachse 7, welche die Schaltwippe 2 in die zwei Endstellungen bringt. Die Radachse 7 sorgt für die Umschaltung der Schaltwippe 2. Sie hat die Form einer Hantel. Im Bereich der Achse wird sie mittels der Achsenfeder 13, einer normalen Druckfeder, aus Ihrer Führung in der Schaltwippe 2 gedrückt. Die paarigen Räder an der Achse müssen dadurch automatisch der Form der Aussparungen 1c im Ventilkörper 1 folgen und zwingen so die Schaltwippe 2 in den jeweiligen Endanschlag für "Geöffnet" bzw. "Geschlossen. Das Ventil kann somit nur einen vollends geschlossenen oder vollends geöffneten Zustand einnehmen.

In der linken unteren Ecke befindet sich im Winkel von beispielsweise 40° zum Durchgangskanal 1a eine durchgehende Stufenbohrung und eine keilförmige Aussparung zur Aufnahme einer Sicherungsvorrichtung, bestehend aus dem Entsicherungsbolzen 10, einer Entsicherungsbolzenfeder 15 und einer Sicherungsplatte 14.
Der quaderförmige Ventilkörper 1 hat durch die Bohrungen und Rundungen eine sichelförmige Hauptoberfläche. Auf dieser schmalen Kante befinden sich mehrere, beispielsweise 6 Stück, zylinderförmige Stifte. Zum Beispiel mittels Ultraschall-Punktschweisstechnik kann so der Ventilkörper 1 mit dem Ventildeckel 3 verbunden werden. Der Ventildeckel 3 schliesst somit die gesamte Ventiltechnik nach aussen hin ab.

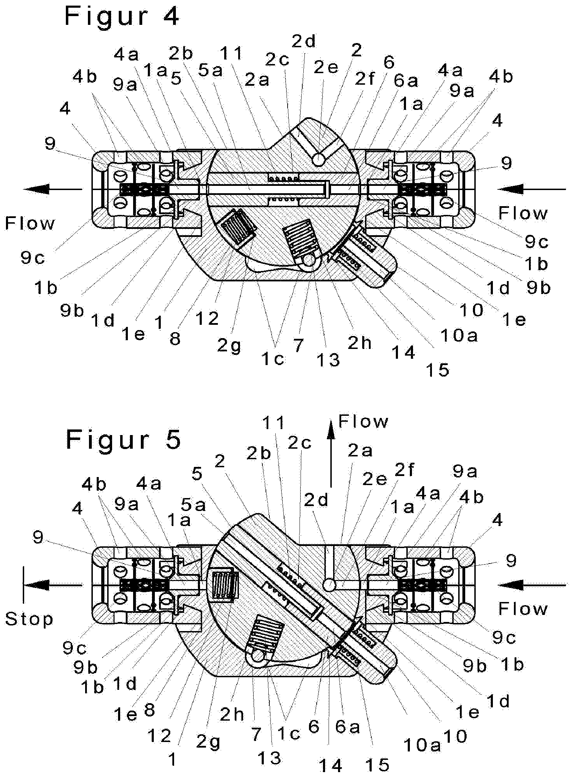

Die Schaltwippe 2 hat die Form eines Zylinders geringer Höhe, dessen Zylindermantel den Ventilanschlüssen zugewandt ist sowie einen Zylinderboden und -deckel, die parallel zu den Seitenwänden 3 des Ventilkörpers ausgerichtet sind. Der Zylindermantel der Schaltwippe 2 weist eine Aussparung mit Flächen 2a und 2b auf, die sich über knapp einen Viertel seines Umfangs erstreckt. Die Aussparung in der Schaltwippe 2 besteht somit aus zwei zum Zylinderboden und -deckel senkrecht stehenden Flächen 2a und 2b, die den Winkel der Aussparung, beispielsweise 140° bilden. Die Fläche 2b ist in der geöffneten Position des Ventils 22 bündig mit dem Ventilkörper 1 während die Fläche 2a aus dem Ventilkörper hervorragt. In der geschlossenen Position ist die Fläche 2a bündig mit dem Körper, während Fläche 2b hervorragt. Zudem liegt die Fläche 2a nahe dem testikulären Einlassende des Ventils (das Einlassende des Ventils ist in Figur 2- 4 mit dem in das Ventil hinein zeigenden Pfeil "Flow" bezeichnet), wobei sie bei geschlossenem Ventil bündig mit dem Ventilkörper am Einlassende verläuft. Entsprechend liegt die Fläche 2b nahe dem abdominalen Auslassende des Ventils und verläuft bei geöffnetem Ventil bündig mit dem Ventilkörper am Auslassende des Ventils. (Das Auslassende des Ventils ist in Figur 2 und 4 mit dem aus dem Ventil hinaus zeigenden Pfeil "Flow" bezeichnet.)
Die Schaltwippe 2 ist allseitig abgerundet und weist mehrere Bohrungen auf. Davon führt eine erste Bohrung 2c parallel zum Zylinderdeckel und -boden und durch das Zentrum des Zylinders 2 und parallel zur Fläche 2b. Diese Bohrung ermöglicht das Fliessen von Spermien im Fall eines geöffneten Ventils 22 und nimmt dazu Schieberohre mit einem Aussenfalz 5 und ein Schieberohr mit einem Innenfalz 6 auf, welche von einer Druckfeder 11 nach aussen gedrückt werden. Dadurch entsteht aufgrund der Feder und der passend gewölbten Geometrie der äusseren Stirnflächen der Schieberohre 5 und 6 und dessen Bohrungen 5a und 6a zusammen mit den Durchgangsbohrungen 1a im Ventilkörper und den Bohrung 9a im Ventilanschlussstück 9 ein nach aussen abgedichteter Durchgang für die Spermien im geöffneten Zustand des Samenleiterventils 22. Weitere Bohrungen 2d und 2f verlaufen ebenfalls parallel zum Zylinderdeckel und führen von einer ersten Öffnung am Zylindermantel im Bereich des Einlassendes 4a des Ventils axial mit der gegenüberliegenden Stufenbohrung 2g des Ventils und von einer zweiten senkrechten Bohrung in der Fläche 2a ins Innere der Schaltwippe 2, wobei die Bohrungen 2d und 2f sich an einem Schnittpunkt treffen. Eine weitere Bohrung 2e schneidet diesen Schnittpunkt rechtwinklig zu diesen Bohrungen 2d und 2f und fluchtet mit einer Bohrung an der Seite des Ventils 22, die nach aussen führt. Im geschlossenen Zustand des Samenleiterventils 22 fluchtet die Bohrung 2e auch mit einer Bohrung 3a im Ventildeckel 3 und eben dieser genau gegenüberstehender Bohrung in der Wandung des Ventilkörpers 1. Die vorgenannten Bohrungen dienen der Abführung von Spermien nach aussen bzw. in das Innere des Scrotums 19, im Fall des geschlossenen Ventils.
Die beiden Flächen 2a und 2b bilden die Tastflächen für das Umschalten des Ventils 22. Drückt man auf die Fläche 2b für "Geöffnet", dreht sich die Schaltwippe 2 im Ventilkörper 1 um einen vorgegebenen Winkel, beispielsweise 40°, und rastet an dieser Stelle fix und gefedert ein. Ein Druck auf die andere Fläche 2a für "Geschlossen" lässt die Schaltwippe 2 wieder um 40° in die andere Richtung springen. Die Schaltwippe 2 nimmt in einer Sacklochbohrung 2g einen Absperrbolzen 8 und eine Bolzenfeder 12 als Dichtungselemente auf. Die Bohrungsachse der abgestuften Sacklochbohrung 2g für die Aufnahme des Absperrbolzen 8 und der Bolzenfeder 12 verläuft parallel zur Fläche 2a "Geschlossen" und kreuzt rechtwinklig die Hoch- oder Längsachse der zylinderförmigen Schaltwippe 2. Die Schieberohranordnung mit Bohrungen 5a und 6a liegt um den gleichen vorbestimmten Winkel, z.B. 40°, verdreht zur Bohrungsachse für den Absperrbolzen 8 und damit parallel mit der Fläche 2b "Geöffnet".
In einer weiteren Bohrung 2h, welche diametral zur Fläche 2a und 2b eingebracht ist, befindet sich die Radachsenfeder 13, welche die Radachse 7 aus der Schaltwippe 2 herausdrückt und für den einrastenden Effekt sorgt. Die Radachse 7 wird dazu in genau bemessenen Führungsnuten in der Schaltwippe 2 im Bereich um die Bohrung 2h geführt. Zudem sind an dieser Seite der Schaltwippe 2 im Ventilkörper zwei Aussparungen 1c angeordnet, in denen die gefederte Radachse 7 mit geringem Spiel und genau bemessenen Verschiebeweg aufgenommen wird. Die Aussparungen sind an ihren den Anschlussstücken zugewandten Seiten kreissegmentförmig ausgebildet, sodass die Radachse 7 dort arretiert wird. An ihren, der Mitte des Ventils zugewandten Seite weisen die Aussparungen 1c je eine nach oben zur Schaltwippe 2 hin gewinkelte Fläche auf, die sich in der Mittellinie des Ventils 22 treffen.

Dabei wird die Radachse 7 in der geschlossenen Ventilstellung in der auslassseitigen Aussparung 1c aufgenommen (in der Figur auf der linken Seite), wobei sie bei Umschaltung des Ventils 22 in die einlass-seitige Aussparung 1c bewegt wird und dort aufgenommen wird (in der Figur auf der rechten Seite). Das Schieberohr mit Aussenfalz 5 und das Schieberohr mit Innenfalz 6, die in der Schaltwippe 2 montiert werden, sind so gefertigt, dass sie ineinander gesteckt werden können und dabei mittels der Druckfeder 11, einer normalen Druckfeder, auseinandergedrückt werden. Das bewirkt ein Abdichten des Durchgangskanals 5a und 6a zum Durchgangskanal 1a des Ventilkörpers 1. Die nach aussen ragenden Enden der Schieberohre besitzen dazu noch zusätzlich eine gewölbte Geometrie, die der Form der grossen Stufenbohrung im Ventilkörper 1 entspricht. Ist das Ventil geöffnet, verläuft die Schieberohranordnung in gleicher Achse mit dem Ventildurchgang. Wird das Ventil durch eine -40° Drehung der Schaltwippe 2 geschlossen, zeigt die Öffnung eines Schieberohrs nach aussen ins Freie. Das andere Schieberohr rastet rechts unten in die Bohrung der Sicherungsplatte 14 ein. Soll das Ventil geschlossen werden, muss erst der Entsicherungsbolzen von unten aussen gegen den Widerstand einer Entsicherungsbolzenfeder 15 gedrückt werden. Erst dann kann gleichzeitig das Ventil geöffnet werden. Der Entsicherungsbolzen 10 weist, wie die Schieberohre, eine Durchgangsbohrung 10a auf und erlaubt eine Vereinnahmung der Hohlräume durch die umgebene Körperflüssigkeit der Schieberohre im geschlossenen Zustand des Ventils. Eine Sicherungsplatte 14 ist ein mit einer Trapezform versehenes, nach innen gewölbtes und mit einer Stufenbohrung ausgeführtes Plättchen und wird bei der Montage des Ventils nach dem Einführen des Entsicherungsbolzens 10 und der Entsicherungsbolzenfeder 15 in die passende Aussparung im Ventilkörper 1 geschoben. Die Entsicherungsbolzenfeder 15 kann als mehrteilige Tellerfeder oder als normale Druckfeder ausgeführt werden. Mit der Variante der Tellerfedern lässt sich so ein Klick-Effekt erzielen, der das Entsichern des Entsicherungsbolzens 10 anzeigt.
Die eigentliche Abdichtung des Spermienflusses, durch das geschlossene Ventil, wird vom Absperrbolzen 8 gewährleistet. Beim Schliessen des Ventils schiebt sich dieser vor das abdominale Durchgangsloch im Ventilinneren, also die Auslassöffnung des Ventils im Bereich dessen Auslassendes. Entsprechend ist das Auslassende in Figur 5 im Fall der Absperrung mit einem Pfeil "Stop" bezeichnet, wobei die Spermien jedoch durch den Abflusskanal 2f, 2e, und 2d abfliessen. Der Absperrbolzen 8 wird mittels der Bolzenfeder 12, einer normalen Druckfeder, gegen die grosse Stufenbohrungswandung des Ventilkörpers gedrückt und hat an der Berührungsfläche mit dem Ventilkörper 1 die gleiche gewölbte Geometrie. Auf diese Art kommt das Samenleiterventil 22 ohne elastische, verschleissende Dichtungsmaterialien aus. Es wird, statt wie sonst bei Ventilen üblich, bei längerer Benutzung nicht undicht. Durch die geringe Reibung im Bereich der Bahn, welcher der Absperrbolzen am Ventilkörper 1 beschreibt, schleifen sich sogar beide Seiten aufeinander ein und werden demzufolge immer dichter. Andererseits ist bei der gegebenen geringen Schaltfrequenz von vermutlich unter 10 Mal innerhalb der lebenslangen Nutzungszeit der zu erwartende Verschleiss zu vernachlässigen.
Für ein Spermium mit einem Kopfdurchmesser von etwa 3,5 Mikrometer ist diese Barriere unüberwindbar. Zumal sich die Spermien im Bereich des Samenleiters noch in einer Säuresperre befinden, also sich nicht selbst bewegen können.

Als Drehachse, genau im Mittelpunkt der Schaltwippe 2, dient je ein zylinderförmiger Zapfen (nicht dargestellt) auf beiden Seiten der Schaltwippe 2, welcher aus der Oberfläche des Zylinderdeckels bzw. -bodens herausragt. Die Zapfen passen in die (nicht dargestellten) Sacklochbohrungen des Ventilkörpers auf der einen Seite und in die Sacklochbohrung des Ventildeckels 3 auf der anderen Seite.
Der Ventildeckel 3 ist im Prinzip ein Spiegelbild der grossen Seitenwand des Ventilkörpers. Er verschliesst die ganze Mechanik nach der Montage nach aussen hin ab. Er besitzt dazu noch mehrere, zum Beispiel 6 Löcher 3b zur Aufnahme entsprechender Stifte am Ventilkörper 1, um mit diesem passgenau verschweisst werden zu können. Alternativ könnte der Ventildeckel 3 aber auch mit dem Ventilkörper 1 verschraubt oder mittels Einrasttechnik mit dem Ventilkörper 1 verbunden werden.

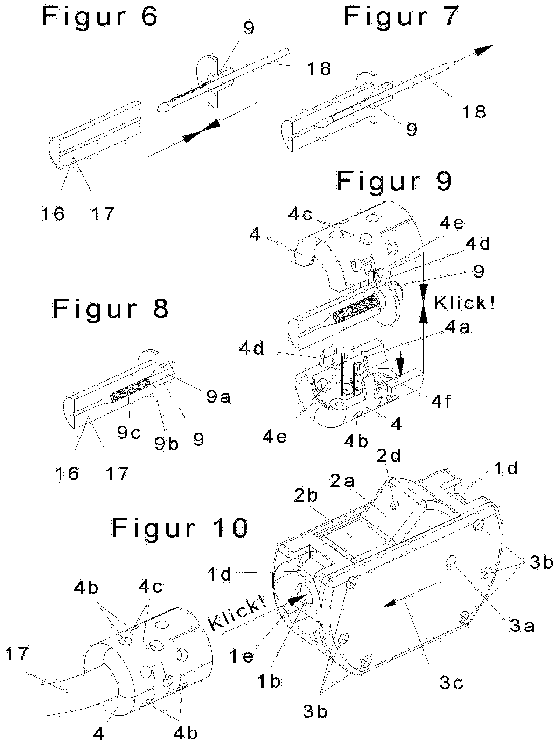

Wie in den Figuren 6-9 dargestellt, besitzen die Ventilanschlussstücke 9 einen grossen Flansch 9b, welcher bei der Implantation von der zweiteiligen Ventilkappe 4 in dessen Nuten 4a eingeklemmt wird. Auf der Anschlussseite Richtung Ventilkörper 1 befindet sich ein zylinderförmiger Stutzen, der bei der Montage der Einheit bestehend aus Samenleiter 16 / 17, Ventilanschluss 9 und der zweiteiligen Ventilkappe 4 entsprechend der Figur 9 in die Bohrung 1b des Ventilkörpers 1 gesteckt werden kann. Auf der Gegenseite befindet sich zur Aufnahme des jeweiligen Samenleiterendes ein dünnwandiges Röhrchen 9c. Das Ventilanschlussstück 9 besitzt eine durchgehende Öffnung, die durch Stutzen, Flansch und Röhrchen führt und den Fluss der Spermien gewährleistet. In einer Ausführung der Erfindung (nicht dargestellt) weist das Röhrchen 9c mehrere feine konusförmige Abstufungen wie bei einer Schlauchtülle auf. Bei der Implantation stehen mehrere fein abgestufte unterschiedliche Innendurchmesser der Röhrchen zwecks Anpassung an den Samenleiter zur Verfügung. In einer weiteren Ausführung der Erfindung ist das Röhrchen 9c mit einer gitterartigen Struktur in der Mantelfläche versehen. Wie bei einem Stent ist die Gitterstruktur vor dem Implantieren etwas zusammengedrückt. Ausserdem ist dieses Ende noch mit einer dünnen Silikonschicht (nicht dargestellt), beispielsweise einem Silikonschlauch oder einem ähnlichen elastischen inerten Material ummantelt, um die Abdichtung der inneren Samenleiterschleimhaut (Mucosa) mit dem Ventilanschlussstück 9 im Bereich von 9c bis 9b zu gewährleisten. Nach dem Einführen der Ventilanschlussstücke 9 in die Samenleiter 16, 17 werden diese von innen her auf das Nenninnenmass aufgeweitet. Dies geschieht, indem ein längliches, nagelartiges Element 18 mit keilförmig geformtem Kopf aus dem Ventilanschlussstück 9 herausgezogen wird.

So lässt sich das Ventilanschlussstück 9 mit minimalem Aussendurchmesser leicht in das kleine Lumen des Samenleiters stecken. Mittels einer nicht Einführen der Ventilanschlussstücke 9 in die Samenleiter 16, 17 werden diese von innen her auf das Nenninnenmass aufgeweitet. Dies geschieht, indem ein längliches, nagelartiges Element 18 mit keilförmig geformtem Kopf aus dem Ventilanschlussstück 9 herausgezogen wird.

So lässt sich das Ventilanschlussstück 9 mit minimalem Aussendurchmesser leicht in das kleine Lumen des Samenleiters stecken. Mittels einer nicht dargestellten Spezialzange, die dem Prinzip einer Blindnietzange entspricht, wird Element 18 aus dem Ventilanschlussstück 9 gezogen und weitet das Lumen des Ventilanschlusses im Bereich der Gitterstruktur zusammen mit dem Samenleiter 16, 17 selbst leicht auf.

Eine weitere Variante des Aufweitens der Gitterstruktur des Ventilanschlussstückes 9 ist auch möglich mit dem umgekehrten Prinzip durch das Einpressen eines zylinderförmigen Dorns mit kegelförmiger Spitze anstelle des dargestellten Elements 18 mit Hilfe einer ebenfalls nicht dargestellten ähnlichen Spezialzange.

Die hohlzylinderförmigen Ventilkappen 4 schützen durch ihre wulstige abgerundete Form die Samenleiter 16, 17 vor einem Einstechen der Ventilanschlüsse bei extremen Bewegungen. Die Mantelflächen der Ventilkappen 4 sind ringsherum mit Bohrungen 4b versehen, durch die die Samenleiterenden stets mit dem natürlichen Fluidum des umliegenden Gewebes in Kontakt bleiben und der Stoffwechsel ermöglicht wird. Eine Ventilkappe 4 besteht aus zwei Hälften, die mit Widerhaken 4d und Aussparungen versehen sind. Mit deren Hilfe lassen sich die beiden Hälften zusammen stecken und gleichzeitig das Ventilanschlüsstück 9 darin befestigen.

Zusammengesteckt bilden die beiden Hälften einen Hohlzylinder. Er hat auf der Seite zum Samenleiter 16, 17 hin eine grosse, abgerundete Kante und eine nach innen gerichtete Wulst. Der Innendurchmesser der Wulst sollte dem
Des Weiteren sind in den Hälften Mikronadeln 4e eingearbeitet. Sie sind so angeordnet, dass sie beim Zusammendrücken der Ventilkappenhälften die muskulösen Wandungen des Samenleiters tangential durchstechen und in eingearbeitete Löcher auf der gegenüberliegenden Ventilkappenhälfte 4 einstechen, ohne dabei auf der anderen Seite auszutreten. Dies sorgt für eine sichere Verbindung des Samenleiterventils mit dem Samenleiter 16, 17, ohne die Blutversorgung des Samenleiters durch eine Abbindung oder sonstigen Naht einzuschränken oder zu gefährden. Der grosse Flansch des Ventilanschlussstückes 9 bettet sich dabei in die Innennut 4a der Ventilkappenhälften 4 ein.
Die so entstandene Einheit aus Anschlussstück 9, Samenleiter 16, 17 und Ventilkappenhälften 4 wird abschliessend in das Samenleiterventil gesteckt. Sie rastet dort ebenfalls dank der Widerhaken 4f in die Einrastelemente 1d und der kreisnutförmigen Aussparung 1e des Ventilkörpers 1 ein. Die beschriebenen Schritte in den Figuren 6 bis 10 erfolgen sowohl mit dem abdominalen 17 als auch mit dem testikulären Samenleiterende 16. Dadurch entsteht ein unlösbarer Verbund des Samenleiters mit dem Ventil. Diese Verbindung könnte auch so konstruiert werden, dass sie mittels eines weiteren Spezialwerkzeugs doch wieder lösbar ist.
Vorzugsweise werden für das erfindungsgemässe Samenleiterventil inerte Materialien verwendet. Dadurch bedürfen die Oberflächen der Einzelteile keiner Antihaftbeschichtungen und Medikamente zur Vermeidung von Abstossungsreaktionen des menschlichen Körpers.
In einer Ausführung der Erfindung ist eine auf Einzelteile beschränkte oder auf alle Teile des Samenleiterventils 22 ausgeweitete Oberflächenbehandlung zum vorgenannten Zweck optional gegeben.

Um auf die unterschiedlichen anatomischen Dimensionen der Samenleiter 16, 17 verschiedener Männer reagieren zu können, sind die Ventilanschlüsse 9 und Ventilkappen 4 entsprechend unterschiedlich gross gefertigt. So kann der Chirurg bei der Implantation die passende Grösse wählen. Der Ventilkörper 1 selbst, mit den inliegenden Teilen, könnte dabei immer gleich gross bleiben. Somit eignet sich eine Implantation des erfindungsgemässen Samenleiterventils auch als Alternative für eine Vasovasostomie, wenn beide Samenleiterenden 16,17 eines Samenleiters unterschiedliche Dimensionen aufweisen, wie es sich nach vor Jahren erfolgter Vasektomie oft darstellt.

Mit dem Schliessen des Samenleiterventils ist der Anwender nicht sofort, sondern erst nach Wochen bis Monaten, steril. Dies braucht nicht als Nachteil gelten. Wird z.B. in einer Partnerschaft mit Kinderwunsch die Schwangerschaft erreicht, wird das Ventil geschlossen. Spätestens mit der Geburt des Kindes, also in der Regel etwa nach 9 Monaten nach der Empfängnis, ist der Anwender dann steril. Weitere Kontrazeptiva seitens der Frau oder des Mannes werden nicht mehr benötigt. Erst wenn ein erneuter Kinderwunsch besteht, z.B. nach 2-4 Jahren, werden die Samenleiterventile geöffnet. Dann geht es schneller. So konnten z.B. nach 4 Wochen bzw. schon bei der ersten Ejakulationen nach Implantation des neuentwickelten Samenleiterventils Spermien im Ejakulat nachgewiesen werden, obwohl zuvor eine einjährige Sterilität vorlag.

Bis auf das Kondom haben alle Kontrazeptiva für den Mann, einschliesslich der medikamentösen Mittel und auch die hiermit beschriebene Erfindung, den Nachteil, dass der Anwender nicht sofort nach dem Aktivieren der Absperrfunktion oder der Einnahme des Medikaments steril ist. Es können sich noch wochen- bis monatelang Spermien in den, der jeweiligen Vorrichtung nachfolgenden Organe, wie der Vorsteherdrüse 25 (Prostata)und der Bläschendrüse 26 (Glandulae vesiculosa) aufhalten und mit einer Ejakulation während eines Koitus hervorgebracht eine unerwünschte Befruchtung auslösen.
Deshalb ist es ratsam die gewünschte Sterilität zuvor mittels eines Spermiogrammes wissenschaftlich bestätigen zu lassen.
Ausserdem schützen diese Methoden nicht vor Infektionskrankheiten.

### Bezugszeichenliste

*1 Ventilkörper*
   1a Durchgangskanal im Ventilkörper
   1b Bohrung im Ventilkörper für Aufnahme Ventilanschlussstück 9
   1c Aussparungen im Ventilkörper zur Arretierung der Radachse 7
   1d Aussparung im Ventilkörper zur Einrastung der Ventilkappe 4
   1e Aussparung im Ventilkörper zur Aufnahme der Ventilkappe 4
2 Schaltwippe
   2a Schaltfläche der Schaltwippe zum Verschliessen des Ventils 22
   2b Schaltfläche der Schaltwippe zum Öffnen des Ventils 22
   2c Bohrung der Schaltwippe zur Aufnahme der Schieberohre 5+6
   2d Bohrung in der Schaltfläche 2a zur Abführung der Spermien
   2e Bohrung durch die Schaltwippe zur Abführung der Spermien
   2f Bohrung in der Schaltwippenkante zur Abführung der Spermien
   2g Bohrung der Schaltwippe zur Aufnahme des Absperrbolzens 8
   2h Bohrung und Aussparung zur Aufnahme der Radachse 7 und Feder 13
3 Ventildeckel
   3a Bohrung im Ventildeckel zur Abführung der Spermien
   3b Bohrung / Schweisspunkt zum Verbinden Ventildeckel und -körper
   3c Kennzeichnung der Fliessrichtung am Ventildeckel
4 Ventilkappe
   4a Nut in der Ventilkappe zur Aufnahme des Flansches 9b des Ventilanschlussstücks 9
   4b Bohrungen in der Ventilkappe als Verbindung des eingeschlossenen Samenleiters nach aussen
   4c Bohrungen in der Ventilkappe zur Aufnahme der Mikronadel 4e der gegenseitigen Ventilkappenhälfte
   4d Widerhaken zum Ineinanderrasten der Ventilkappenhälften
   4e Mikronadeln in den Ventilkappenhälften
   4f Widerhaken zum Einrasten der Ventilkappe 4 in den Ventilkörper 1
5 Schieberohr mit Aussenfalz
   5a Durchgangskanal im Schieberohr mit Aussenfalz
6 Schieberohr mit Innenfalz
   6a Durchgangskanal im Schieberohr mit Innenfalz
7 Radachse
8 Absperrbolzen
9 Ventilanschlussstück
   9a Durchgangskanal im Ventilanschlussstück
   9b Flansch am Ventilanschlussstück
   9c Durchgangsröhrchen mit Gitterstruktur am Ventilanschlussstück
10 Entsicherungsbolzen
   10a Durchgangskanal im Entsicherungsbolzen
11 Schieberohrfeder
12 Bolzenfeder
13 Radachsenfeder
14 Sicherungsplatte
15 Entsicherungsbolzenfeder
16 Samenleiterende testikulär
17 Samenleiterende abdominal
18 nagelartiges Element zum Aufweiten des Durchgangsröhrchens 9c
19 Hodensack (Scrotum)
20 Hoden (Testis)
21 Nebenhoden (Epididymis)
22 Samenleiterventil (komplett / allgemein)
23 Bläschendrüse (Glandulae vesiculosae)
24 Vorsteherdrüse (Prostata)

## Patentansprüche

1. Implantierbares Samenleiterventil (22) zur Kontrazeption zur Anwendung beim Mann oder männlichen Tier zur Regulierung des Spermienflusses im abdominalen Samenleiter (17) oder Vas deferens innerhalb des Scrotums (19) mit einem Ventil (22), das mittels Ventilanschlussstücken (9) an das testikuläre und abdominale Ende eines durchtrennten Samenleiters (16, 17) befestigbar ist, und einen manuellen Schalter zur Veränderung des Zustands des Ventils aufweist, dessen Schaltzustand von aussen durch Palpation feststellbar ist und zwischen einem geöffnetem und geschlossenem Zustand veränderbar ist und das Samenleiterventil (22) einen Durchgangskanal (1a, 5a, 6a, 9a) aufweist, der im geöffneten Zustand des Samenleiterventils (22) vom Ventilanschlussstück (9) am testikulären Samenleiter (16) zum Ventilanschlussstück (9) am abdominalen Ende des Samenleiters (17) führt und im geschlossenen Ventilzustand des Samenleiterventils (22) an dem Ende des Durchgangskanals (1a) absperrbar ist, das dem abdominalen Samenleiterende (17) zugewandt ist
**dadurch gekennzeichnet, dass**
das Samenleiterventil (22) mindestens einen Abflusskanal (2d, 2e, 2f, 3a) aufweist, der im geschlossenen Zustand des Ventils (22) von dem Ventilanschlussstück (9) am testikulären Ende des Samenleiters (16) aus dem Samenleiterventil (22) nach aussen und in den Körper des Nutzers führt.

2. Implantierbares Samenleiterventil (22) nach Anspruch 1
**dadurch gekennzeichnet, dass**
der Schalter als Schaltwippe (2) ausgebildet ist.

3. Implantierbares Samenleiterventil (22) nach Anspruch 1
**dadurch gekennzeichnet, dass**
der Durchgangskanal (5a, 6a) im Bereich des Schalters (2) in einem Schieberohr mit einem Innenfalz (5) und einem Aussenfalz (6) und einer Druckfeder (11) angeordnet ist.

4. Implantierbares Samenleiterventil (22) nach Anspruch 1
**dadurch gekennzeichnet, dass**
die Schaltwippe (2) einen Absperrbolzen (8) mit Feder (12) aufweist zur Absperrung des Durchgangskanals (1a) am Auslassende des Samenleiterventils (22).

5. Implantierbares Samenleiterventil (22) nach Anspruch 1
**dadurch gekennzeichnet, dass**
die Schaltwippe (2) eine Radachse (7) mit Feder (13) und Aussparungen (2h) zur einrastenden Aufnahme der Radachse (7) aufweist.

6. Implantierbares Samenleiterventil (22) nach Anspruch 1
**dadurch gekennzeichnet, dass**
ein Entsicherungsbolzen (10) mit einer Entsicherungsbolzenfeder (15) an der Schaltwippe (2) angeordnet ist.

7. Implantierbares Samenleiterventil (22) nach Anspruch 1
**dadurch gekennzeichnet, dass**
der Entsicherungsbolzen (10) Öffnungen aufweist, die aus dem Ventilkörper (1) des Samenleiterventils (22) heraus führen.

8. Implantierbares Samenleiterventil (22) nach Anspruch 1
**dadurch gekennzeichnet, dass**
das Samenleiterventil (22) Ventilanschlusstücke (9) zur Aufnahme der Samenleiterenden (16, 17) aufweist mit einem Röhrchen (9c), dessen Wandung aussen konusförmige Abstufungen aufweist.

9. Implantierbares Samenleiterventil (22) nach Anspruch 1
**dadurch gekennzeichnet, dass**
das Samenleiterventil (22) Ventilanschlusstücke (9) zur Aufnahme der Samenleiterenden (16, 17) aufweist mit einem Röhrchen (9c), dessen Wandung mit einer Gitterstruktur versehen ist.

10. Implantierbares Samenleiterventil (22) nach Anspruch 8 oder 9
**dadurch gekennzeichnet, dass**
die Ventilanschlussstücke (9) in Ventilkappen (4) des Samenleiterventils (22) aufgenommen sind.

11. Implantierbares Samenleiterventil (22) nach Anspruch 10
**dadurch gekennzeichnet, dass**
in den Ventilkappen (4) Mikronadeln (4e) angeordnet sind zur Befestigung der Ventilkappen (4) an den Wandungen der Samenleiter (16, 17).

12. Implantierbares Samenleiterventil (22) nach Anspruch 11
**dadurch gekennzeichnet, dass**
die Ventilkappen (4) Bohrungen (4b) aufweisen, die nach aussen führen und einen Kontakt der Samenleiter (16, 17) mit Körperflüssigkeit gewährleisten.

13. Implantierbares Samenleiterventil (22) nach Anspruch 10
**dadurch gekennzeichnet, dass**
die Ventilkappen (4) Innennuten (4a) aufweisen zur Aufnahme der Ventilanschlussstücke (9).

14. Implantierbares Samenleiterventil (22) nach Anspruch 10
**dadurch gekennzeichnet, dass**
die Ventilkappen (4) aus je zwei zusammensteckbaren Hälften bestehen.

15. Implantierbares Samenleiterventil (22) nach Anspruch 8 oder 9
**dadurch gekennzeichnet, dass**
die Ventilanschlussstücke (9) des Samenleiterventil (22) aus Metall-Legierungen oder Kunststoff oder aus einer Kombination beider Werkstoffarten gefertigt sind.

16. Implantierbares Samenleiterventil (22) nach Anspruch 9
**dadurch gekennzeichnet, dass**
die Gitterstruktur des Röhrchens (9c) ganz oder teilweise mit Silikon oder einem elastischen inerten Material ummantelt ist.

17. Implantierbares Samenleiterventil (22) nach einem der vorangehenden Ansprüche 1-16
**dadurch gekennzeichnet, dass**
Einzelteile des Samenleiterventils oder das gesamte Samenleiterventil (22) mit Medikamenten oder Antihaftbeschichtungen beschichtet ist.

## Claims

1. An implantable vas deferens valve (22) for contraception for use by the male human or male animal to regulate sperm flow in the abdominal vas deferens (17) or vas deferens within the scrotum (19), having a valve (22) that can be fastened by means of valve connectors (9) to the testicular and abdominal end of a severed vas deferens (16, 17), and having a manual switch for changing the state of the valve, the switching state of which is detectable from the outside by palpation and changeable between an open and closed state and the vas deferens valve (22) has a through-passage (1a, 5a, 6a, 9a), which in the opened state of the vas deferens valve (22) leads from the valve connector (9) on the testicular vas deferens (16) to the valve connector (9) at the abdominal end of the vas deferens (17), and in the closed valve state of the vas deferens valve (22) can be shut off at the end of the through-passage (1a) which faces the abdominal vas deferens end (17), **characterized in that**
the vas deferens valve (22) has at least one outflow channel (2d, 2e, 2f, 3a), which in the closed state of the valve (22) leads from the valve connector (9) at the testicular end of the vas deferens (16) out of the vas deferens valve (22) to the outside and into the body of the user.

2. Implantable vas deferens valve (22) according to claim 1, **characterized in that** the switch is designed as a rocker switch (2).

3. Implantable vas deferens valve (22) according to claim 1, **characterized in that** the through-passage (5a, 6a) in the region of the switch (2) is arranged in a sliding tube with an inner fold (5) and an outer fold (6) and a compression spring (11).

4. Implantable vas deferens valve (22) according to claim 1, **characterized in that** the rocker switch (2) has a shut-off pin (8) with spring (12) for shutting off the through-passage (1a) at the outlet end of the vas deferens valve (22).

5. Implantable vas deferens valve (22) according to claim 1, **characterized in that** the rocker switch (2) has a wheel axle (7) with spring (13) and recesses (2h) for receiving the wheel axle (7) in a latching manner.

6. Implantable vas deferens valve (22) according to claim 1, **characterized in that** a release bolt (10) with a release bolt spring (15) is arranged on the rocker switch (2).

7. Implantable vas deferens valve (22) according to claim 1, **characterized in that** the release bolt (10) has openings leading out of the valve body (1) of the vas deferens valve (22).

8. Implantable vas deferens valve (22) according to claim 1, **characterized in that** the vas deferens valve (22) has valve connectors (9) for receiving the vas deferens ends (16, 17) with a tube (9c), the wall of which has outside conical graduations.

9. Implantable vas deferens valve (22) according to claim 1, **characterized in that** the vas deferens valve (22) has valve connectors (9) for receiving the vas deferens ends (16, 17) with a tube (9c), the wall of which is provided with a lattice structure.

10. Implantable vas deferens valve (22) according to claim 8 or 9, **characterized in that** valve connectors (9) are accommodated in valve caps (4) of the vas deferens valve (22).

11. Implantable vas deferens valve (22) according to claim 10, **characterized in that** microneedles (4e) are arranged in the valve caps (4) for attachment of the valve caps (4) on the walls of the vas deferens (16, 17).

12. Implantable vas deferens valve (22) according to claim 11, **characterized in that** the valve caps (4) have bores (4b) which lead to the outside and ensure contact of the vas deferens (16, 17) with body fluid.

13. Implantable vas deferens valve (22) according to claim 10, **characterized in that** the valve caps (4) have internal grooves (4a) for receiving the valve connectors (9).

14. Implantable vas deferens valve (22) according to claim 10, **characterized in that** the valve caps (4) consist of two mutually attachable halves each.

15. Implantable vas deferens valve (22) according to claim 8 or 9, **characterized in that** the valve connectors (9) of the vas deferens valve (22) are made of metal alloys or plastic or of a combination of both types of material.

16. Implantable vas deferens valve (22) according to claim 9, **characterized in that** the lattice structure of the tube (9c) is completely or partially coated with silicone or an elastic inert material.

17. Implantable vas deferens valve (22) according to one of the preceding claims 1 to 16, **characterized in that** individual parts of the vas deferens valve or the entire vas deferens valve (22) are coated with drugs or non-stick coatings.

## Revendications

1. Clapet de canal déférent implantable (22) destiné à la contraception pour une application chez l'homme ou l'animal mâle en vue de réguler le flux de sperme dans le canal déférent abdominal (17) ou vas deferens à l'intérieur du scrotum (19) avec un clapet (22) qui peut être fixé au moyen de pièces de raccordement de clapet (9) aux extrémités testiculaire et abdominale d'un canal déférent sectionné (16, 17), et qui présente un commutateur manuel pour modifier l'état du clapet, dont l'état de commutation est vérifiable et modifiable entre un état ouvert et fermé de l'extérieur par palpation, et le clapet de canal déférent (22) présentant un canal traversant (1a, 5a, 6a, 9a) qui, lorsque le clapet de canal déférent (22) est à l'état ouvert, conduit de la pièce de raccordement de clapet (9) au canal déférent testiculaire (16) à la pièce de raccordement de clapet (9) à l'extrémité abdominale du canal déférent (17) et, lorsque le clapet de canal déférent (22) est à l'état fermé, peut être coupé à l'extrémité du canal traversant (1a) qui est tournée vers l'extrémité abdominale du canal déférent (17), **caractérisé en ce que** le clapet de canal déférent (22) présente au moins un canal d'évacuation (2d, 2e, 2f, 3a) qui, lorsque le clapet (22) est fermé, conduit de la pièce de raccordement de clapet (9) à l'extrémité testiculaire du canal déférent (16) hors du clapet de canal déférent (22) vers l'extérieur et dans le corps de l'utilisateur.

2. Clapet de canal déférent implantable (22) selon la revendication 1, **caractérisé en ce que** le commutateur est réalisé sous la forme d'un commutateur à bascule (2).

3. Clapet de canal déférent implantable (22) selon la revendication 1, **caractérisé en ce que** le canal traversant (5a, 6a) est disposé dans la zone du commutateur (2) dans un tube coulissant présentant une feuillure intérieure (5), une feuillure extérieure (6) et un ressort de compression (11).

4. Clapet de canal déférent implantable (22) selon la revendication 1, **caractérisé en ce que** le commutateur à bascule (2) présente un boulon d'arrêt (8) avec un ressort (12) pour couper le canal traversant (1a) à l'extrémité de sortie du clapet de canal déférent (22).

5. Clapet de canal déférent implantable (22) selon la revendication 1, **caractérisé en ce que** le commutateur à bascule (2) présente un axe de roue (7) avec un ressort (13) et des évidements (2h) destinés à recevoir avec encliquetage l'axe de roue (7).

6. Clapet de canal déférent implantable (22) selon la revendication 1, **caractérisé en ce qu'**un boulon de déblocage (10) avec un ressort de boulon de déblocage (15) est disposé sur le commutateur à bascule (2).

7. Clapet de canal déférent implantable (22) selon la revendication 1, **caractérisé en ce que** le boulon de déblocage (10) présente des ouvertures qui conduisent hors du corps de clapet (1) du clapet de canal déférent (22).

8. Clapet de canal déférent implantable (22) selon la revendication 1, **caractérisé en ce que** le clapet de canal déférent (22) présente des pièces de raccordement de clapet (9) destinées à recevoir les extrémités de canal déférent (16, 17), qui comportent un petit tube (9c) dont la paroi présente extérieurement des gradins coniques.

9. Clapet de canal déférent implantable (22) selon la revendication 1, **caractérisé en ce que** le clapet de canal déférent (22) présente des pièces de raccordement de clapet (9) destinées à recevoir les extrémités de canal déférent (16, 17), qui comportent un petit tube (9c) dont la paroi est pourvue d'une structure réticulaire.

10. Clapet de canal déférent implantable (22) selon la revendication 8 ou 9, **caractérisé en ce que** les pièces de raccordement de clapet (9) sont reçues dans des capuchons de clapet (4) du clapet de canal déférent (22).

11. Clapet de canal déférent implantable (22) selon la revendication 10, **caractérisé en ce que** des micro-aiguilles (4e) destinées à fixer les capuchons de clapet (4) aux parois du canal déférent (16, 17) sont disposées dans les capuchons de clapet (4).

12. Clapet de canal déférent implantable (22) selon la revendication 11, **caractérisé en ce que** les capuchons de clapet (4) présentent des trous (4b) qui conduisent vers l'extérieur et assurent un contact des canaux déférents (16, 17) avec le fluide corporel.

13. Clapet de canal déférent implantable (22) selon la revendication 10, **caractérisé en ce que** les capuchons de clapet (4) présentent des rainures intérieures (4a) destinées à recevoir les pièces de raccordement de clapet (9).

14. Clapet de canal déférent implantable (22) selon la revendication 10, **caractérisé en ce que** les capuchons de clapet (4) sont constitués chacun de deux moitiés emboîtables.

15. Clapet de canal déférent implantable (22) selon la revendication 8 ou 9, **caractérisé en ce que** les pièces de raccordement de clapet (9) du clapet de canal déférent (22) sont réalisées en alliages métalliques ou en matière plastique ou en une combinaison des deux types de matériau.

16. Clapet de canal déférent implantable (22) selon la revendication 9, **caractérisé en ce que** la structure réticulaire du petit tube (9c) est enveloppée entièrement ou partiellement de silicone ou d'un matériau élastique inerte.

17. Clapet de canal déférent implantable (22) selon l'une des revendications précédentes 1 à 16, **caractérisé en ce que** des parties du clapet de canal déférent ou la totalité du clapet de canal déférent (22) est/sont revêtue(s) de médicaments ou de revêtements anti-adhérents.
